# EUROPEAN PATENT SPECIFICATION

(11) **EP 1 261 617 B1**
(45) Date of publication and mention of the grant of the patent: **31.05.2006**
(21) Application number: 01920251.4
(22) Date of filing: 08.03.2001
(51) Int. Cl.: C07H 21/00, C07H 21/04, C12Q 1/68, C12M 1/36

(54) **METHODS FOR ASSAY AND DETECTION ON A MICROARRAY**
TEST- UND NACHWEISVERFAHREN FÜR EINEN MIKROARRAY
METHODE D'ESSAI ET DE DETECTION SUR UN MICRO-RESEAU

(30) Priority: 08.03.2000 US 187681 P
(43) Date of publication of application: 04.12.2002
(73) Proprietor: DATASCOPE INVESTMENT CORP., Montvale, N.J. 07645 (US)
(72) Inventor: GETTS, Robert C. c/o Datascope Investment Corp., Montvale, NJ 07645 (US)
(74) Representative: Bergstrand, Mikael Gudmundsson
(86) International application number: PCT/US2001/007477
(87) International publication number: WO 2001/066555

(56) References cited:
- US-A- 5 124 246
- US-A- 5 175 270
- US-A- 5 849 481
- US-A- 6 004 755
- US-A- 6 027 890
- NILSEN ET AL.: 'Dendritic nucleic acid structures' JOURNAL OF THEORETICAL BIOLOGY vol. 187, 1997, pages 273 - 284, XP002940183

## Description

### Field of the Invention

The present invention relates to DNA microarrays, more particularly to methods for detection and assay of a nucleic acid sequence sample by hybridization on a microarray.

### Background of the Invention

Changes in gene expression patterns or in a DNA sequence can have profound effects on biological functions. Such variations in gene expression may result in altered physiologic and pathologic processes. Developing DNA technologies are providing rapid and cost-effective methods for identifying gene expression and genetic variations on a large-scale level. One high-speed technology useful for DNA analysis is the DNA microarray which includes a plurality of distinct DNA or gene probes (i.e., polynucleotides) distributed spatially, and stably associated with a substantially planar substrate such as a plate of glass, silicon or nylon membrane. Such microarrays have been developed and are used in a range of applications such as analyzing a sample for the presence of gene variations or mutations (i.e. genotyping), or for patterns of gene expression, while performing the equivalent of thousands of individual "test-tube" experiments carried out in a short period of time.

All microarrays operate on a similar principle: a substantially planar substrate such as a glass coverslide is coated with a grid of tiny spots of about 20 to 100 microns in diameter each spot or feature contains millions of copies of a short sequence of DNA or nucleotides; and a computer keeps track of each sequence at a predetermined feature. To make an analysis, messenger RNA (mRNA) is extracted from a sample of cells. Using enzymes, millions of copies of the mRNA molecules are reproduced. Copies of complementary DNA (cDNA) are generated from the mRNA through reverse transcription. The cDNA copies are tagged with a marker or label such as a fluorescent marker and broken up into short fragments. The tagged fragments are washed over the microarray and left overnight, to allow the tagged fragments to hybridize with the DNA attached to the microarray.

After hybridization, the features on the microarray that have paired with the fluorescent cDNA emit a fluorescent signal that can be viewed with a microscope or detected by a computer. In this manner, one can learn which sequences on the microarray match the cDNA of the test sample. Although there are occasional mismatches, the employment of millions of probes in each spot or feature ensure fluorescence is detected only if the complementary cDNA is present The more intense the fluorescent signal, (i.e. the brighter the spot) the more matching cDNA was present in the cell.

One area in which the microarrays are useful is in gene expression analysis. In gene expression analysis utilizing microarrays, an array of "probe" oligonucleotides is contacted with a nucleic acid sample of interest, i.e. target, such as cDNA generated from mRNA extracted from a particular tissue type. Contact is carried out under hybridization conditions and unbound nucleic acid is then removed. The resultant pattern of hybridized nucleic acid provides information regarding the genetic profile of the sample tested. Genetic profile is meant to include information regarding the types of nucleic acids present in the sample, (e.g. the types of genes to which they are complementary, as well as the copy number of each particular nucleic acid in the sample). Gene expression analysis may be use in a variety of applications, including, for example, the identification of novel expression of genes, the correlation of gene expression to a particular phenotype, screening for disease predisposition, and identifying the effect of a particular agent on cellular gene expression, such as in toxicity testing.

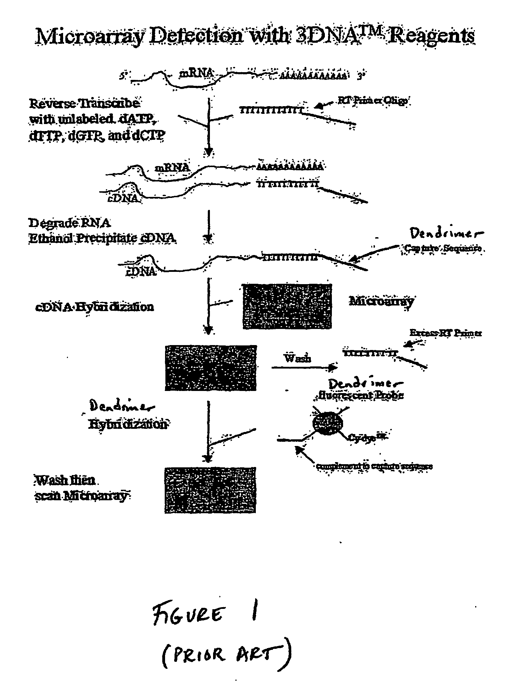

A prior art example of a method of preparing a nucleic acid sequence sample for detecting and assaying a gene expression sequence is shown in Figure 1 and described as follows. Using known methods, a plurality of gene probes are affixed or printed on the surface of a microarray such as by robotic or laser lithographic processes. The complementary DNA (cDNA) is prepared from a mRNA sample comprised of total RNA or poly(A)⁺ RNA, along with a large quantity of nucleotide bases (deoxynucleotide triphosphate, DNTP), enzymes and reverse transcription (RT) primer oligonucleotides with capture sequence portions appended thereto. The newly formed cDNA is then isolated from the mRNA sample and precipitated with ethanol. The cDNA is then suspended in a cDNA hybridization buffer for hybridizing the cDNA to the microarray with the complementary gene probes and incubated overnight. Following hybridization of the cDNA to the prepared microarray, the microarray is washed to remove any excess RT primer oligonucleotide. A mixture containing labeled dendritic nucleic acid molecules is then prepared.

Dendritic nucleic acid molecules, or dendrimers are complex, highly branched molecules, comprised of a plurality of interconnected natural or synthetic monomeric subunits of double-stranded DNA. Dendrimers are described in greater detail in Nilsen et al., Dendritic Nucleic Acid Structures, J. Theor. Biol., 187, 273-284 (1997).

Dendrimers comprise two types of single-stranded hybridization "arms" on the surface which are used to attach two key functionalities. A single dendrimer molecule may have at least one hundred arms of each type on the surface. One type of arm is used for attachment of a specific targeting molecule to establish target specificity and the other is used for attachment of a label or marker. The molecules that determine the target and labeling specificities of the dendrimer are attached either as oligonucleotides or as oligonucleotide conjugates. Using simple DNA labeling, hybridization, and ligation reactions, a dendrimer molecule may be configured to act as a highly labeled, target specific probe.

The prepared mixture is formulated in the presence of a suitable buffer to yield a dendrimer hybridization mixture containing dendrimers with fluorescent labels attached to one type of ''arm", and with oligonucleotides attached to another type of "arm", complementary to the capture sequences of the RT primer bound cDNA fragments. The dendrimer hybridization mixture containing the dendrimer molecules, is then added to the microarray and incubated overnight to generate a hybridization pattern. Subsequent to the dendrimer-to-cDNA hybridization, the microarray is washed to purge any excess unhybridized dendrimers. The microarray is scanned to detect the signal generated by the label to enable gene expression analysis of the hybridization pattern. One of the drawbacks using this method includes the undue time and labor required to prepare the sample and to perform the assay including the hybridization and washing steps.

It would be highly desirable to significantly reduce the amount of time and labor expended in preparation of the sample and performing the assay without sacrificing desirable attributes such as sensitivity, low background "noise", and minimal "false positives". It would be a significant advance in the art of gene expression detection microarrays to further provide a method which significantly reduces the complexity and the steps needed to prepare gene samples and the assay for gene expression analysis, and which can be carried out using conventional laboratory reagents, equipment and techniques.

### Summary of the Invention

The present invention relates generally to methods for assay and detection on a microarray. The present invention provides significant reduction in the time and labor required to produce a hybridization pattern for obtaining information about the genetic profile of the target nucleic acid sample, and the source from which the sample was obtained. The present invention further provides a microarray with excellent sensitivity and low background "noise" and minimal "false positives". The method of the present invention may be used in a range of applications.

In one aspect of the present invention, a method is provided for assay and detection on a microarray which comprises the steps of:
1) contacting a microarray having thereon a plurality of features each containing a first particular first nucleotide sequence with a mixture containing:
   a) a first component comprising a cDNA reagent obtained from mRNA of a target sample, said cDNA having a capture sequence; and
   b) a second component comprising a dendrimer having at least one first arm containing a label capable of emitting a detectable signal and at least one second arm having a second nucleotide sequence complementary to the capture sequence;
2) mixing the first and second components at a temperature and for a time sufficient to enable the first component to bind to the second component; and
3) incubating this mixture with said microarray to enable the first nucleotide sequence to bind to the first component, wherein such binding results in the feature emitting the detectable signal.

In another aspect of the invention, there is provided a method for assay and detection on a microarray which comprises the steps of:
1) incubating a mixture including:
   i) a first component comprising a cDNA reagent obtained from mRNA of a target sample, said cDNA having a capture sequence; and
   ii) a second component comprising a dendrimer having at least one first arm containing a label capable of emitting a detectable signal and at least one second arm having a second nucleotide sequence complementary to the capture sequence,
   at a first temperature and for a time sufficient to induce the first component to bind to the second component and form a prehybridized cDNA-dendrimer complex;
2) contacting a microarray having thereon a plurality of features each containing a particular first nucleotide sequence with said mixture; and
3) incubating the microarray and the prehybridized cDNA-dendrimer complex at a second temperature and for a time sufficient to induce the prehybridized cDNA-dendrimer complex to bind to the first nucleotide sequence, wherein such binding results in the feature emitting the detectable signal whereby a hybridization pattern is generated on the microarray.

In another aspect of the present invention, a method is provided for assay and detection on a microarray which comprises the steps of:
1) contacting a microarray having thereon a plurality of features each containing a first particular first nucleotide sequence with a mixture containing:
   a) a first component comprising a cDNA reagent obtained from mRNA of a target sample, said cDNA having a capture sequence; and
   b) a second component comprising a dendrimer having at least one first arm containing a label capable of emitting a detectable signal and at least one second arm having a second nucleotide sequence complementary to the capture sequence;
2) mixing the first and second components at a temperature and for a time sufficient to enable the first component to bind to the second component; and
3) incubating the microarray and the prehybridized cDNA-dendrimer complex at a second temperature and for a time sufficient to induce the prehybridized cDNA-dendrimer complex to bind to the first nucleotide sequence, wherein such binding results in the feature emitting the detectable signal whereby a hybridization pattern is generated on the microarray.

In another aspect of the invention, there is provided a method for assay and detection on a microarray which comprises the steps of:
1) incubating a mixture including:
   i) a first component comprising a cDNA reagent obtained from mRNA of a target sample, said cDNA having a capture sequence; and
   ii) a second component comprising a dendrimer having at least one first arm containing a label capable of emitting a detectable signal and at least one second arm having a second nucleotide sequence complementary to the capture sequence,
   at a first temperature and for a time sufficient to induce the first component to bind to the second component and form a prehybridized cDNA-dendrimer complex;
2) mixing the first and second components at a temperature and for a time sufficient to enable the first component to bind to the second component; and
3) incubating the microarray and the prehybridized cDNA-dendrimer complex at a second temperature and for a time sufficient to induce the prehybridized cDNA-dendrimer complex to bind to the first nucleotide sequence, wherein such binding results in the feature emitting the detectable signal whereby a hybridization pattern is generated on the microarray.

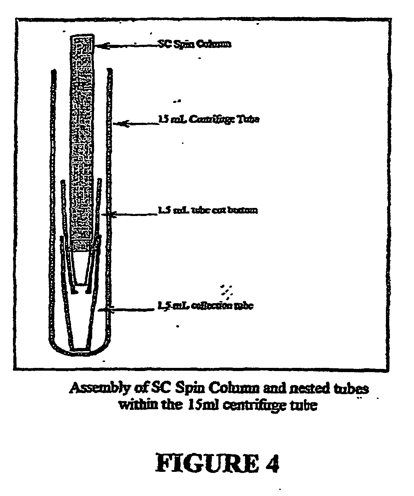

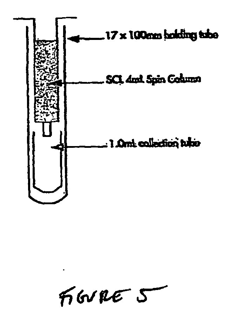

In additional embodiments of the invention, such cDNA is purified using a spin column (e.g. such as that of Figure 4 or 5), or using a hybridization chamber or station.

### Brief Description of the Drawings

The following drawings in which like reference characters indicate like parts are illustrative of embodiments of the invention and are not to be construed as limiting the invention as encompassed by the claims forming part of the application.
Figure 1 is a schematic representation of prior art steps for preparing a microarray for detection and assay of a nucleic acid sequence sample;
Figure 2 is a schematic representation of a method for preparing a microarray for detection and assay of a nucleic acid sequence sample in one embodiment of the present invention;
Figure 3 is a schematic representation of a method for preparing a microarray for detection and assay of a nucleic acid sequence sample in another embodiment of the present invention; and
Figure 4 is a cross sectional view of a spin column assembly used in accordance with the method represented in Figure 3.
Figure 5 is a cross sectional view of an additional embodiment of a spin column for use in accordance with the present invention.

### Detailed Description of the Invention

The present invention is generally directed to a method for preparing a nucleic acid sequence sample for detection and assay on a microarray in a manner that provides a significant reduction in time and effort typically required in assaying a sample on a microarray. The method of the present invention provides the advantage of preparing the nucleic acid sequence sample in a shorter period of time, using fewer steps while providing the sensitivity, low background "noise", and minimal "false positives" required for laboratory and clinical use. The cost effective and efficient manner by which the nucleic acid sequence samples are prepared and by which the method of the present invention can be implemented using conventional laboratory techniques, equipment and reagents, makes them especially suitable for research and clinical use.

In the methods of the present invention, an array of DNA or gene probes fixed or stably associated with the surface of a substantially planar substrate is contacted with a sample oftarget nucleic acids under hybridization conditions sufficient to produce a hybridization pattern of complementary probe/target complexes. A variety of different microarrays which may be used, are known in the art. The hybridized samples of nucleic acids are then targeted by labeled dendrimer probes and hybridized to produce a detectable signal corresponding to a particular hybridization pattern. The individual labeled dendrimer probes hybridized to the target nucleic acids are all capable of generating the same signal of known intensity. Thus, each positive signal in the microarray can be "counted" in order to obtain quantitative information about the genetic profile of the target nucleic acid sample.

Before the present invention is further described, it is to be understood that the invention is not limited to the particular embodiments of the invention described below, as variations of the particular embodiments may be made and still fall within the scope of the appended claims. It is also to be understood that the terminology employed is for the purpose of describing particular embodiments, and is not intended to be limiting. Instead, the scope of the present invention will be established by the appended claims.

The DNA or gene probes of the microarrays which are capable of sequence specific hybridization with target nucleic acid may be polynucleotides or hybridizing analogues or mimetics thereof, including, but not limited to, nucleic acids in which the phosphodiester linkage has been replaced with a substitute linkage group, such as phophorothioate, methylimino, methylphosphonate, phosphoramidate, guanidine and the like, nucleic acids in which the ribose subunit has been substituted, e.g. hexose phosphodiester, peptide nucleic acids, and the like. The length of the probes will generally range from 10 to 1000 nucleotides. In some embodiments of the invention, the probes will be oligonucleotides having from 15 to 150 nucleotides and more usually from 15 to 100 nucleotides. In other embodiments the probes will be longer, usually ranging in length from 150 to 1000 nucleotides, where the polynucleotide probes may be single or double stranded, usually single stranded, and may be PCR fragments amplified from cDNA or cloned genes. The DNA or gene probes on the surface of the substrates will preferably correspond to, but are not limited to, known genes of the physiological source being analyzed and be positioned on the microarray at a known location so that positive hybridization events may be correlated to expression of a particular gene in the physiological source from which the target nucleic acid sample is derived. Because of the manner in which the target nucleic acid sample is generated, as described below, the microarrays of gene probes will generally have sequences that are complementary to the non-template strands of the gene to which they correspond.

The substrates with which the gene probes are stably associated may be fabricated from a variety of materials, including plastic, ceramic, metal, gel, membrane, glass, and the like. The microarrays may be produced according to any convenient and conventional methodology, such as preforming the gene probes and then stably associating them with the surface of the support or growing the gene probes directly on the support. A number of different microarray configurations and methods for their production are known to those of skill in the art, one of which is described in Science, 283, 83, 1999.

The term "label" is used herein to refer to agents that are capable of providing a detectable signal, either directly or through interaction with one or more additional members of a signal producing system. Labels that are directly detectable and may find use in the present invention include fluorescent labels such as fluorescein, rhodamine, BODIPY, cyanine dyes (e.g. from Amersham Pharmacia), Alexa dyes (e.g. from Molecular Probes, Inc.) fluorescent dye phosphoramidites, and the like; and radioactive isotopes, such as ³² S, ³² P, ³ H, etc.; and the like. Examples of labels that provide a detectable signal through interaction with one or more additional members of a signal producing system include capture moieties that specifically bind to complementary binding pair members, where the complementary binding pair members comprise a directly detectable label moiety, such as a fluorescent moiety as described above. The label is one which preferably does not provide a variable signal, but instead provides a constant and reproducible signal over a given period of time.

The present invention further utilizes dendritic nucleic acid molecules, or dendrimers as label probe molecules. Dendrimers are complex, highly branched molecules, comprised of a plurality of interconnected natural or synthetic monomeric subunits of double-stranded DNA. Dendrimers are described in Nilsen et al., Dendritic Nucleic Acid Structures, J. Theor.-Biol., 187, 273-284 (1997), the entire content of which is incorporated herein by reference. Further information regarding the structure and production of dendrimers is disclosed in U.S. Pat. Nos. 5,175,270, 5,484,904, and 5,487,973.

Dendrimers comprise two types of single-stranded hybridization "arms" on the surface which are used to attach two key functionalities. A single dendrimer molecule may have at least one hundred arms of each type. One type of arm is used for attachment to targeting molecules (e.g. a capture sequence) to establish target specificity and the other is used for attachment of a label or marker. The molecules that determine the target and labeling specificities of the dendrimer are attached either as oligonucleotides or as oligonucleotide conjugates. Using simple DNA labeling, hybridization, and ligation reactions, the dendrimer probes may be configured to act as a highly labeled, target specific reagent

To prepare fluorescent labeled dendrimer, the complementary sequences to the capture sequence on a Cap01 RT primer and a Cap02 RT primer (both also from Oligos, etc.) are ligated, separately, to the purified dendritic core material as prepared by the previously described methods (see Nilson et al., supra, and U.S. Pat. Nos. '270, '904, and'973, supra.). In the preferred embodiments, the Cap01 RT primer is a Cy3® RT primer and the Cap02 RT primer is a Cy5® RT primer; however, the invention is not limited to those two preferred embodiments. Thirty nucleotide long oligonucleotides complementary to the outer arms of a four-layer dendrimer having a 5' Cy3® or Cy5® (in the preferred embodiments) or Cap01 and Cap02 more generally (the Cy3®, Cy5®, Cap01, and Cap02 being available from Oligos etc., Inc., Wilsonville, OR), are then synthesized. The Cy3® and Cy5® oligonucleotides are then hybridized and covalently cross-linked to the outer surface of the corresponding dendrimers, respectively. Excess capture and fluorescent labeled oligonucleotides are then removed through techniques such as size exclusion chromatography.

The concentration of dendrimer is determined by measuring the optical density of the purified material at 260 nm on a UV/Vis spectrometer. The fluorescence is measured at optimal signal/noise wavelengths using a fluorometer (FluoroMax, SPEX Industries). In the preferred embodiment, using Cy3 and Cy5, Cy3 is excitable at 542 nm and the emission measured at 570 nm; Cy5 is excitable at 641 nm and the emission at 676 nm.

In the present invention, the use of dendrimer probes significantly reduces the amount of sample RNA needed to generate an assay while increasing sensitivity due to the dendrimers' superior signal amplification capability. By reducing the amount of RNA required for an assay, the amount of RT primer may be likewise reduced for improved signal generation as discussed below. The reduced RT primer amount also reduces the number of washes needed during assay preparation. The present invention also reduces the hybridization process into a single step for increased sensitivity and ease of use, and significant reduction in processing time. The hybridization speed and efficiency is greatly enhanced by first hybridizing the cDNA to the dendrimer probes before hybridizing the cDNA to the microarray. This single-step hybridization process also reduces the number of hybridization buffers to one by eliminating the use of a cDNA hybridization buffer (50% formamide, 10% dextran sulfate, 1X Denhardt's solution, 0.2% N-Lauroyl sarcosine, 250 µg/mL sheared salmon sperm DNA, 2X SSC, 20 mM Tris pH 7.5, and double distilled water).

The target nucleic acid will generally be DNA that has been reverse transcribed from RNA derived from a naturally occurring source, where the RNA may be selected from the group consisting of total RNA, poly(A)⁺mRNA, amplified RNA and the like. The initial mRNA source may be present in a variety of different samples, where the sample will typically be derived from a physiological source. The physiological source may be derived from a variety of eukaryotic sources, with physiological sources of interest including sources derived from single celled organisms such as yeast and multicellular organisms, including plants and animals, particularly mammals, where the physiological sources from multicellular organisms may be derived from particular organs or tissues of the multicellular organism, or from isolated cells derived therefrom. In obtaining the sample RNAs to be analyzed from the physiological source from which it is derived, the physiological source may be subjected to a number of different processing steps, where such known processing steps may include tissue homogenation, cell isolation and cytoplasmic extraction, nucleic acid extraction and the like. Methods of isolating RNA from cells, tissues, organs or whole organisms are known to those of ordinary skill in the art and are described, for example, in Maniatis et al., Molecular Cloning: A Laboratory Manual, 2^{nd} ed., Cold Spring Harbor Laboratory Press, 1989, and in Ausubel et al., Current Protocols in Molecular Biology, John Wiley & Sons, Inc., 1998.

The sample mRNA is reverse transcribed into a target nucleic acid in the form of a cDNA, by hybridizing an oligo(dT) primer, or RT primer, to the mRNA under conditions sufficient for enzymatic extension of the hybridized primer. The primer will be sufficiently long to provide for efficient hybridization to the mRNA tail, where the region will typically range in length from 10 to 25 nucleotides, usually 10 to 20 nucleotides, and more usually from 12 to 18 nucleotides.

Recognizing that applications typically require the use of sequence specific primers, the standard primers as used in the present invention further include "capture sequence" nucleotide portions. The preferred capture sequences referred to herein are Cap01 RT primer capture sequence (Oligos etc., Inc, Wilsonville, OR) or Cap 02 primer capture sequence (Oligos etc., Inc, Wilsonville, OR) and further preferably are Cy3® RT primer capture sequence (Oligos etc., Inc, Wilsonville, OR) or Cy5® RT primer capture sequence (Oligos etc., Inc, Wilsonville, OR), and are represented below.
Cap 01 RT primer capture sequence:
5' - ggC CTC ACT gCg CgT CTT Ctg TCC CgC C - 3'; and
Cap02 RT primer capture sequence:
5' - CCT gTT gCT CTA TTT CCC gTg Ccg CTC Cgg T - 3'.

For custom primers, the above capture sequences should be attached to the 5' end of the corresponding custom oligonucleotide primer. In this manner, the custom primer replaces the standard RT primer. Since the present invention is devised for use with the standard RT primer, some modifications may be required when substituting a custom primer. Such modifications are known to those of ordinary skill in the art and may include adjusting the amount and mixture of primers based on the amount and type of RNA sample used. The primer carries a capture sequence comprised of a specific sequence of nucleotides, as described above. The capture sequence is complementary to the oligonucleotides attached to the arms of dendrimer probes which further carry at least one label. Such complementary oligonucleotides may be acquired from any outside vendor and may also be acquired as labeled moieties. The label may be attached to one or more of the oligonucleotides attached to the arms of the dendrimer probe, either directly or through a linking group, as is known in the art. In the preferred embodiment, the dendrimer probes are labeled by hybridizing and crosslinking Cy3® or Cy5® labeled oligonucleotides (in the preferred embodiment) to the dendrimer arms. The Cy3® or Cy5® labeled dendrimers are complementary to the Cap01 or Cap02 RT primer capture sequences, respectively.

In generating the target nucleic acid sample, the primer is contacted with the mRNA in the presence of a reverse transcriptase enzyme, and other reagents necessary for primer extension under conditions sufficient for inducing first strand cDNA synthesis, where additional reagents include: dNTPs; buffering agents, e.g. Tris.Cl; cationic sources, both monovalent and divalent, e.g. KCl, MgCl₂ ; RNAase inhibitor and sulfhydril reagents, e.g. dithiothreitol; and the like. A variety of enzymes, usually DNA polymerases, possessing reverse transcriptase activity can be used for the first strand cDNA synthesis step. Examples of suitable DNA polymerases include the DNA polymerases derived from organisms selected from the group consisting of a thermophilic bacteria and archaebacteria, retroviruses, yeasts, Neurosporas, Drosophilas, primates and rodents. Suitable DNA polymerases possessing reverse transcriptase activity may be isolated from an organism, obtained commercially or obtained from cells which express high levels of cloned genes encoding the polymerases by methods known to those of skill in the art, where the particular manner of obtaining the polymerase will be chosen based primarily on factors such as convenience, cost, availability and the like. The order in which the reagents are combined may be modified as desired.

In one preferred embodiment, the cDNA synthesis protocol involves combining from about 0.25 to 1 µg of total RNA or from about 12.5 to 50 ng of poly(A)⁺mRNA, with about 0.2 pmole of RT primer (0.2 pmole) and RNase free water for a final volume of about 10 µL to yield RNA-RT primer mix. The RNA-RT primer mixture is then mixed and microfuged to collect the contents at the bottom of the microfuge tube. The RNA-RT primer mixture is then heated to 80° C for ten minutes and immediately transferred to ice. In a separate microfuge tube on ice, mix together about 4µL 5X RT buffer, 1 µL dNTP mix, 4 µL RNase free water and 1 µL of 200 Unit reverse transcriptase enzyme. Gently mix and microfuge briefly to collect the contents at the bottom of the microfuge tube to yield a reaction mixture. Mix the RNA-RT primer mixture with the reaction mixture and then incubate at about 42°C for a period of time sufficient for forming the first strand cDNA primer extension product, which usually takes about 2 hours.

The mixture comprising the cDNA ortarget nucleic acid subsequent to formation, may be further purified to remove any excess RT primers which may still remain after completion of the reverse transcription process. The excess RT primer would bind to the dendrimer probes resulting in reduced signal strength and intensity and thus reduced assay sensitivity. Although this step is optional, the purification of the cDNA mixture tends to improve the signal strength in the microarray resulting in improved signal generation of the hybridization pattern. The amount of RT primer affects the quality of the assay since excess RT primer can diminish signal strength and resolution. The excess RT primers may be removed from the cDNA mixture by any suitable means including the use of a spin column assembly, QIAquick® PCR Purification Kit (Qiagen, Valencia, CA), or a hybridization chamber or station, or so forth. Spin column assemblies are known devices used to separate one or more components from a mixture through centrifugal means.

Preferably, the excess RT primers may be removed via a conventional spin column assembly shown in Figure 4, or in an alternate embodiment, using the spin column of Figure 5. The spin column media is composed of a size exclusion resin core which comprises a plurality of resin pores distributed therethrough. The resin pores are sufficiently large to capture the excess RT primer, and permits cDNA to pass into the void volume. To remove excess RT primer, the cDNA containing mixture is placed into a holding tube at one end of the spin column where the spin column and mixture are subjected to high centrifugal force for a period of time. The mixture diffuses through the column and exits at an opposite end into a collecting receptacle. The resulting eluate collected in the receptacle comprises the purified cDNA probe.

In performing the methods of the present invention, a quantity of a labeled dendrimer probe is added to the purified cDNA probe eluate along with a hybridization buffer under temperature conditions which induces hybridization between the dendrimer probe and the target cDNA. In particular, the mixture is incubated at a first pre-hybridization temperature and for a sufficient time to allow the dendrimer probes to attach to the cDNA. The preferred range for the first prehybridization temperature where a formamide-free hybridization buffer is used, is from about 45 to 60°C, and preferably at 55 °C. Where a formamide-containing hybridization buffer is used, the preferred range of the pre-hybridization temperature is dependent upon the percent content of formamide where the temperature is reduced by 1°C for each 2% formamide present from the standard formamide-free buffer temperature range. The mixture is preferably incubated for about 15 to 20 minutes to allow the cDNA to hybridize with the dendrimer probes to yield a pre-hybridization mix.

The pre-hybridization mix is then added to the microarray and incubated at a second hybridization temperature and for a sufficient time to allow the cDNA to bind to the microarray. The preferred range for the second hybridization temperature where a formamide-free hybridization buffer is used, is from about 42 to 60°C. Where a formamide-containing hybridization buffer is used, the preferred range of the pre-hybridization temperature is dependent upon the percent content of formamide where the temperature is reduced by 1°C for each 2% formamide present from the standard formamide-free buffertemperature range. Preferably, the pre-hybridization mix and the microarray is incubated at the second temperature overnight in a humidified chamber.

Under such initial conditions, the capture sequence of the cDNA is able to pre-hybridize with the complement attached to the dendrimer probe before the cDNA binds to the gene probe of the microarray. The target cDNA attached to the dendrimer probe, is then contacted with the microarray under conditions sufficient to permit hybridization of the target cDNA to the DNA or gene probe on the microarray. The resulting mixture is incubated overnight for complete hybridization. Suitable hybridization conditions are well known to those of skill in the art and reviewed in Maniatis et al., supra, where conditions may be modulated to achieve a desire specificity in hybridization. It is noted that any suitable hybridization buffers may be used in the present invention. In one preferred form, the hybridization buffer composition may comprise 0.25 M NaPO₄, 4.5% SDS, 1 mM EDTA, and 1X SSC. In another preferred form, the hybridization buffer composition may comprise 40% formamide, 4X SSC, and 1% SDS.

Following the hybridization step, where unhybridized dendrimer probe-cDNA complexes are capable of emitting a signal during the detection step, a washing step is employed where the unhybridized complexes are purged from the microarray, thus leaving behind a visible, discrete pattern of hybridized cDNA-dendrimer probes bound to the microarray. A variety of wash solutions and protocols for their use are known to those of skill in the art and may be used. The specific wash conditions employed will necessarily depend on the specific nature of the signal producing system that is employed, and will be known to those of skill in the art familiar with the particular signal producing system employed.

The resultant hybridization pattern of labeled cDNA fragments may be visualized or detected in a variety of ways, with the particular manner of detection being chosen based on the particular label of the cDNA, where representative detection means include scintillation counting, autoradiography, fluorescence measurement, calorimetric measurement, light emission measurement and the like.

Following hybridization and any washing step(s) and/or subsequent treatments, as described above, the resultant hybridization pattern is detected. In detecting or visualizing the hybridization pattern, the intensity or signal value of the label will be not only be detected but quantified, by which is meant that the signal from each spot of the hybridization will be measured.

Following detection or visualization, the hybridization pattern can be used to determine quantitative and qualitative information about the genetic profile of the labeled target nucleic acid sample that was contacted with the microarray to generate the hybridization pattern, as well as the physiological source from which the labeled target nucleic acid sample was derived. From this data, one can also derive information about the physiological source from which the target nucleic acid sample was derived, such as the types of genes expressed in the tissue or cell which is the physiological source, as well as the levels of expression of each gene, particularly in quantitative terms. Where one uses the subject methods in comparing target nucleic acids from two or more physiological sources, the hybridization patterns may be compared to identify differences between the patterns. Where microarrays in which each of the different probes corresponds to a known gene are employed, any discrepancies can be related to a differential expression of a particular gene in the physiological sources being compared. Thus, the subject methods find use in differential gene expression assays, where one may use the subject methods in the differential expression analysis of: diseased and normal tissue, e.g. neoplastic and normal tissue, different tissue or subtissue types; and the like.

### EXAMPLE 1

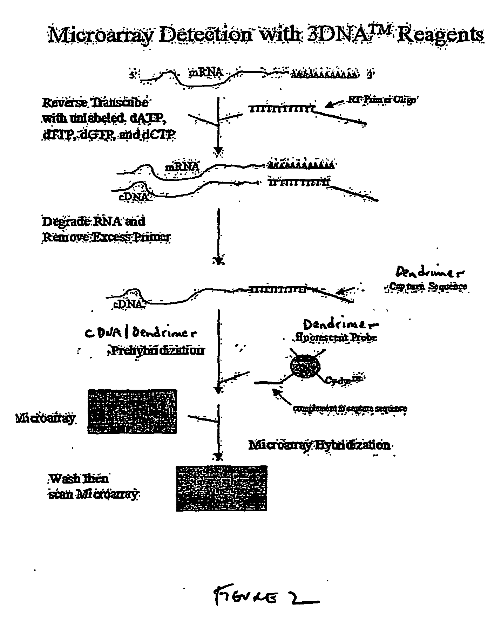

With reference to Figure 2, a method for detection and assay on a microarray is described below.

### Microarray Preparation

A microarray was prepared as directed by the manufacturer or by customary procedure protocol. The nucleic acid sequences comprising the DNA or gene probes were amplified using known techniques in polymerase chain reaction, then spotted onto glass slides, and processed according to conventional procedures.

### Preparation and Concentration of Target Nucleic Acid Sequences Sample, or cDNA

The target nucleic acid sequences, or cDNA was prepared from total RNA or poly(A)+RNA extracted from a sample of cells. It is noted that for samples containing about 10 to 20 µg of total RNA or 500-1000 ng of poly(A)⁺ RNA, ethanol precipitation is not required and may be skipped, because the cDNA is sufficiently concentrated to perform the microarray hybridization. In a microfuge tube, 0.25 to 5 µg of total RNA or 12.5 to 500 ng of poly(A)⁺ RNA was added with 3 µL of Cy3® or Cy5® RT primer (0.2 pmole) and RNase free water for a total volume of 10µL to yield a RNA-RT primer mixture. The resulting mixture was mixed and microfuged briefly to collect contents in the bottom of the microfuge tube. The collected contents was then heated to 80°C for about ten (10) minutes and immediately transferred to ice. In a separate microfuge tube on ice, 4 µL of 5X RT buffer, 1 µL of dNTP mix, 4 µL RNase free water, and 1 µL of reverse transcriptase enzyme (200 Units) were combined to yield a reaction mixture. The reaction mixture was gently mixed and microfuged briefly to collect contents in the bottom of the microfuge tube. 10 µL of the RNA-RT primer mixture and 10 µL of the reaction mixture, was mixed briefly and incubated at 42°C for two hours. The reaction was terminated by adding 3.5µL of 0.5 M NaOH/50mM EDTA to the mixture. The mixture was incubated at 65°C for ten (10) minutes to denature the DNA/RNA hybrids and the reaction was neutralized with 5 µL of 1 M Tris-HCl, pH 7.5. 38.5 µL of 10 mM Tris, pH 8.0, 1 mM EDTA was then added to the neutralized reaction mixture. (The above steps may be repeated replacing the 3 µL of Cy3® RT primer (0.2 pmole) with 3 µL of Cy5® RT primer (0.2 pmole) for preparing dual channel expression assays whereby the prepared Cy3® and Cy5® cDNA mixture are mixed together with 10 µL of 10 Tris, pH 8.0, 1 mM EDTA, to yield a reaction mixture for processing in the following steps.)

2 µL of a carrier nucleic acid (10mg/mL linear acrylamide) was added to the neutralized reaction mixture for ethanol precipitation. 175 µL of 3M ammonium acetate was added to the mixture and then mixed. Then, 625 µL of 100% ethanol was added to the resulting mixture. The resulting mixture was incubated at -20°C for thirty (30) minutes. The sample was centrifuged at an acceleration rate greater than 10,000 g for fifteen (15) minutes. The supernatant was aspirated and then 330 µL of 70 % ethanol was added to the supernatant, or cDNA pellet. The cDNA pellet was then centrifuged at an acceleration rate greater than 10,000 g for 5 minutes, was then remove. The cDNA pellet was dried (i.e., 20-30 minutes at 65° Celsius).

### Hybridization of cDNA/Dendrimer Probe Mixture to Microarray

The DNA hybridization buffer was thawed and resuspended by heating to 65°C for ten (10) minutes. The hybridization buffer comprised of 40% formamide. The buffer was mixed by inversion to ensure that the components were resuspended evenly. The heating and mixing was repeated until all of the material was resuspended. A quantity of competitor DNA was added as required (e.g. COT-1-DNA, and polydA). The cDNA was resuspended in 5.0 µL of sterile water.

In a first embodiment, single channel analysis, 2.5 µL of one type of 3DNA® reagent (Genisphere, Inc., Montvale, NJ) (Cy3 or Cy5) was added to the resuspended cDNA along with 12.5 µL of a DNA hybridization buffer (containing 40% formamide). In an alternative embodiment, for dual channel analysis, 2.5 µL of two types of 3DNA® reagents, Cy3 and Cy5 specifically labeled dendrimers, were added to the resuspended cDNA along with 10 µl of a DNA hybridization buffer. In a further embodiment of multiple channel analysis (with three or more channels), 2.5 µL of three or more types of 3DNA® reagents, Cy3, Cy5, and one or more prepared using another label moiety, were added to the resuspended cDNA along with 10µL of a DNA hybridization buffer.

For larger hybridization buffer volumes, additional DNA hybridization buffer may be added to the required final volume. It is noted that hybridization buffer volumes greater than 35 µL may also require additional 3DNA® reagents.

The DNA hybridization buffer mixture was incubated at about 50°C for about 15 to 20 minutes to allow for prehybridization of the cDNA to the 3DNA® reagents. The prehybridized mixture was then added to the microarray and then incubated overnight at 55°C. At this stage the cDNA was hybridized to the gene probes.

### Post Hybridization Wash

The microarray was briefly washed to remove any excess dendrimer probes. First, the microarray was washed for 10 minutes at 55° C with 2X SSC buffer, 0.2%SDS. Then the microarray was washed for 10 minutes at room temperature with 2X SSC buffer. Finally the microarray was washed for 10 minutes at room temperature with 0.2X SSC buffer.

### Signal Detection

The microarray was then scanned as directed by the scanner's manufacturer for detecting, analyzing, and assaying the hybridization pattern.

### EXAMPLE 2

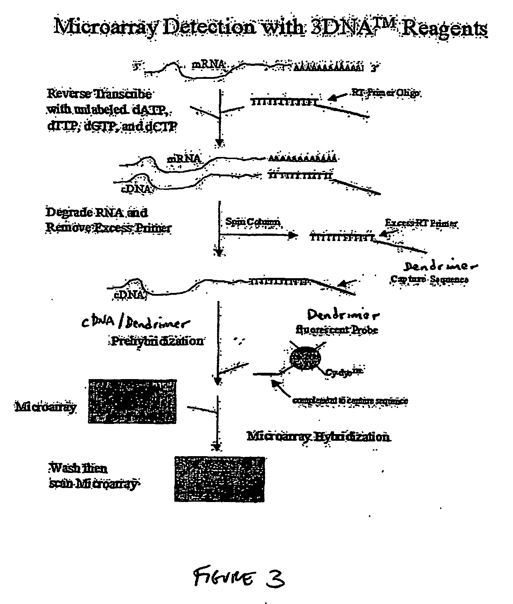

With reference to Figure 3, a method for detection and assay on a microarray is described below. This method includes the use of a spin column assembly (e.g. of Figure 4 or Figure 5) for reducing protocol time and number of steps, and for increasing signal strength.

### Microarray Preparation

A microarray was prepared as directed by the manufacturer or by customary protocol procedures. The nucleic acid sequences comprising the DNA or gene probes were amplified using known techniques in polymerase chain reaction, then spotted onto glass slides, and processed according to conventional procedures.

### Preparation and Concentration of Target Nucleic Acid Sequences, or cDNA

The target nucleic acid sequences, or cDNA was prepared from total RNA or poly(A)+RNA extracted from a sample of cells. In a microfuge tube, 0.25 to 5 µg of total RNA or 12.5 to 500 ng of poly(A)⁺ RNA was added with 1 µL of Cy3® or Cy5® RT primer (5 pmole) and RNase free water for a total volume of 10µL to yield a RNA-RT primer mixture. The resulting mixture was mixed and microfuged briefly to collect contents in the bottom of the microfuge tube. The collected contents was then heated to 80°C for about ten (10) minutes and immediately transferred to ice. In a separate microfuge tube on ice, 4 µL of 5X RT buffer, 1 µL of dNTP mix, 4 µL RNase free water, and 1 µL reverse transcriptase enzyme (200 Units) were combined to yield a reaction mixture. The reaction mixture was gently mixed and microfuged briefly to collect contents in the bottom of the microfuge tube. 10 µL of the RNA-RT primer mixture and 10 µL of the reaction mixture was mixed together and incubated at 42°C for two hours. The reaction was terminated by adding 3.5µL of 0.5 M NaOH/50mM EDTA. The mixture was incubated at 65°C for ten (10) minutes to denature the DNA/RNA hybrids. The reaction was neutralized by the addition of 5 µL of 1 M Tris-HCl, pH 7.5 to the mixture. 71 µL of 10 mM Tris, pH 8.0, 1 mM EDTA was added to the neutralized reaction mixture. (The above steps may be repeated replacing the 1 µL of Cy3® RT primer (5 pmole) with 1 µL of Cy5® RT primer (5 pmole) for preparing dual channel expression assays whereby the prepared Cy3® and Cy5® cDNA mixture are mixed together with 42 µL of 10 mM Tris, pH 8.0, 1 mM EDTA, to yield a reaction mixture for processing in the following steps.)

### cDNA Purification: Removal of Excess RT Primer via a SC Spin Column Assembly

With reference to the spin column of Figure 4, the spin column was inverted several times to resuspend the media and to create an even slurry in the column. The top and bottom caps were removed from the spin column. A microfuge tube was obtained and the bottom tip of the microfuge tube, was snipped off or punctured. One end of the spin column was placed into the punctured microfuge tube, then the punctured microfuge tube was placed into a second, intact microfuge tube, or collection tube. The assembled spin column was then placed into a 15 mL centrifuge tube with the microfuge tube end first as shown in Figure 4. The spin column was centrifuged at about 1000 g for about 3.5 minutes after reaching full acceleration. The spin column was checked to ensure that the column was fully drained after centrifugation and that the end of the spin column was above the liquid line in the collection tube. The collection tube contained about 2 to 2.5 mL of clear buffer voided from the spin column. The resin appeared nearly dry in the column barrel, and well packed without distortions or cracks. If the end of the spin column had been immersed in the liquid portion, the spin column would have been discarded and the abovesteps repeated with a fresh spin column. The spin column was at that point, prepared to remove the excess RT primer in the neutralized reaction mixture.

The drained spin column was removed and a new 1.0 mL collection tube was placed on top of the buffer collection tubes already in the 15 mL centrifuge tube. The voided buffer was discarded. The drained spin column was placed into the new collection tube. 100 µL of the neutralized reaction mixture containing the cDNA was loaded directly into the center of the spin column media. The spin column assembly was centrifuged at 1 0,000xg for about 2.5 minutes upon reaching full acceleration. The eluate collected in the new collection tube was then recovered. The original volume +/-10 percent was recovered. The eluate comprised the cDNA probe.

Alternatively, using the spin column of Figure 5, the spin column was inverted several times to resuspend the media and to create an even slurry in the column. The top and bottom caps were removed from the spin column. The spin column was then placed into a 15 mL centrifuge tube as shown in Figure 5. The spin column was centrifuged at about 1000 g for about 3.5 minutes after reaching full acceleration. The spin column was checked to ensure that the column was fully drained after centrifugation and that the end of the spin column was above the liquid line in the collection tube. The collection tube contained about 2 to 2.5 mL of clear buffer voided from the spin column. The resin appeared nearly dry in the column barrel, and well packed without distortions or cracks. If the end of the spin column had been immersed in the liquid portion, the spin column would have been discarded and the above steps repeated with a fresh spin column. The spin column was at that point, prepared to remove the excess RT primer in the neutralized reaction mixture.

The drained spin column was removed and the clear bufferwas emptied. A new 1.0 mL collection tube was placed in the bottom of the 15 mL centrifuge tube. 100 µL of the neutralized reaction mixture containing the cDNA was loaded directly into the center of the spin column media. The spin column assembly was centrifuged at 10,000x g for about 2.5 minutes upon reaching full acceleration. The eluate collected in the new collection tube was then recovered. The original volume +/-10 percent was recovered. The eluate comprised the cDNA probe.

2µL of a carrier nucleic acid (10mg/mL linear acrylamide) was added to the eluate for ethanol precipitation. 250 µL of 3M ammonium acetate was added to the mixture and mix. Then, 875 µL of 100% ethanol was added to the mixture. The resulting mixture was incubated at -20°C for thirty (30) minutes. The sample was centrifuged at an acceleration rate greater than 10,000xg for fifteen (15) minutes. The supernatant was aspirated and 300 µL of 70 % ethanol was added to the supernatant, or the cDNA pellet. The cDNA pellet was then centrifuged at an acceleration rate greater than 10,000x g for 5 minutes. The supernatant was then removed. The cDNA pellet was dried (i.e. 20-30 minutes at 65° Celsius).

### Hybridization of cDNA/Dendrimer Probe Mixture to Microarray

The DNA hybridization buffer was thawed and resuspended by heating to 65°C and maintained at 65°C for ten (10) minutes. The hybridization buffer comprised of 40% formamide. The buffer was mixed by inversion to ensure that the components were resuspended evenly. The heating and mixing was repeated until all the material was resuspended. A quantity of competitor DNA (e.g. COT-1-DNA, and polydA) may be added, if required. The cDNA was resuspended in 5.0 µL of sterile water.

In a first embodiment, single channel analysis, 2.5 µL of one type of 3DNA® reagent (Genisphere, Inc., Montvale, NJ) (Cy3 or Cy5) was added to the resuspended cDNA along with 12.5 µL of a DNA hybridization buffer (containing 40% formamide). In an alternative embodiment, for dual channel analysis, 2.5 µL of two types of 3DNA® reagents, Cy3 and Cy5 specifically labeled dendrimers, were added to the resuspended cDNA along with 10 µl of a DNA hybridization buffer. In a further embodiment of multiple channel analysis (with three or more channels), 2.5 µL of three or more types of 3DNA® reagents, Cy3, Cy5, and one or more prepared using another label moiety and a unique capture sequence, were added to the resuspended cDNA along with 10µL of a DNA hybridization buffer.

For larger hybridization buffer volumes, additional amounts of the DNA hybridization buffer may be added to reach the required final volume. It is also noted that hybridization buffer volumes greater than 35 µL may also require additional 3DNA® reagents. The DNA hybridization buffer mixture was incubated at a temperature of about 50°C for about 15 to 20 minutes to allow for the prehybridization of the cDNA to the 3DNA® reagents or dendrimer probes. At this stage, the dendrimer probes of the 3DNA® reagent hybridized with the capture sequence on the cDNA. After 20 minutes, the DNA hybridization buffer was then added to the microarray. The microarray and the DNA hybridization buffer were covered and incubated overnight in a humidified chamber at a temperature of about 55°C. At this stage, the cDNA was hybridized to the gene probes.

### Post Hybridization Wash

The microarray was briefly washed to remove any excess dendrimer probes. First, the microarray was washed for 10 minutes at 55°C with 2X SSC buffer, containing 0.2%SDS. Then, the microarray was washed for 10 minutes at room temperature with 2X SSC buffer. Finally, the microarray was washed for 10 minutes at room temperature with 0.2X SSC buffer.

### Signal Detection

The microarray was then scanned as directed by the scanner's manufacturer for detecting, analyzing, and assaying the hybridization pattern.

### EXAMPLE 3

### An Alternative Method for Detection and Assay on a Microarray

### Microarray Preparation

A microarray was prepared as directed by the manufacturer or by customary protocol procedures. The nucleic acid sequences comprising the DNA or gene probes were amplified using known techniques in polymerase chain reaction, then spotted onto glass slides, and processed according to conventional procedures.

### Preparation and Concentration of Target Nucleic Acid Sequences, or cDNA

The target nucleic acid sequences, or cDNA was prepared from total RNA or poly(A)+RNA extracted from a sample of cells. In a microfuge tube, 0.25 to 10 µg of total RNA or 250 to 500 ng of poly(A)⁺ RNA was added with 1 µL of Cy3® or Cy5® RT primer (5 pmole) and RNase free water for a total volume of 10µL to yield a RNA-RT primer mixture. The resulting mixture was mixed and microfuged briefly to collect contents in the bottom of the microfuge tube. The collected contents was then heated to 80°C for about ten (10) minutes and immediately transferred to ice. In a separate microfuge tube on ice, 4 µL of 5X RT buffer, 1 µL of dNTP mix, 4 µL RNase free water, and 1 µL reverse transcriptase enzyme (200 Units) were combined to yield a reaction mixture. The reaction mixture was gently mixed and microfuged briefly to collect contents in the bottom of the microfuge tube. 10 µL of the RNA-IRT primer mixture and 10 µL of the reaction mixture was mixed together and incubated at 42°C for two hours. The reaction was terminated by adding 3.5µL of 0.5 M NaOH/50mM EDTA. The mixture was incubated at 65°C for ten (10) minutes to denature the DNA/RNA hybrids. The reaction was neutralized by the addition of 5 µL of 1 M Tris-HCl, pH 7.5 to the mixture. 71 µL of 10 mM Tris, pH 8.0, 1 mM EDTA was added to the neutralized reaction mixture.

### cDNA Purification: Removal of Excess RT Primer via a SC Spin Column Assembly

With reference to the spin column of Figure 4, the spin column was inverted several times to resuspend the media and to create an even slurry in the column. The top and bottom caps were removed from the spin column. A microfuge tube was obtained and the bottom tip of the microfuge tube, was snipped off or punctured. One end of the spin column was placed into the punctured microfuge tube, then the punctured microfuge tube was placed into a second, intact microfuge tube, or collection tube. The assembled spin column was then placed into a 15 mL centrifuge tube with the microfuge tube end first as shown in Figure 4. The spin column was centrifuged at about 1000 g for about 3.5 minutes after reaching full acceleration. The spin column was checked to ensure that the column was fully drained after centrifugation and that the end of the spin column was above the liquid line in the collection tube. The collection tube contained about 2 to 2.5 mL of clear buffer voided from the spin column. The resin appeared nearly dry in the column barrel, and well packed without distortions or cracks. If the end of the spin column had been immersed in the liquid portion, the spin column would have been discarded and the above steps repeated with a fresh spin column. The spin column was at that point, prepared to remove the excess RT primer in the neutralized reaction mixture.

The drained spin column was removed and a new 1.0 mL collection tube was placed on top of the buffer collection tubes already in the 15 mL centrifuge tube. The voided buffer was discarded. The drained spin column was placed into the new collection tube. 100 µL of the neutralized reaction mixture containing the cDNA was loaded directly into the center of the spin column media. The spin column assembly was centrifuged at 10,000x g for about 2.5 minutes upon reaching full acceleration. The eluate collected in the new collection tube was then recovered. The original volume +/- 10 percent was recovered. The eluate comprised the cDNA probe.

Alternatively, using the spin column of Figure 5, the spin column was inverted several times to resuspend the media and to create an even slurry in the column. The top and bottom caps were removed from the spin column. The spin column was then placed into a 15 mL centrifuge tube as shown in Figure 5. The spin column was centrifuged at about 1000 g for about 3.5 minutes after reaching full acceleration. The spin column was checked to ensure that the column was fully drained after centrifugation and that the end of the spin column was above the liquid line in the collection tube. The collection tube contained about 2 to 2.5 mL of clear buffer voided from the spin column. The resin appeared nearly dry in the column barrel, and well packed without distortions or cracks. If the end of the spin column had been immersed in the liquid portion, the spin column would have been discarded and the above steps repeated with a fresh spin column. The spin column was at that point, prepared to remove the excess RT primer in the neutralized reaction mixture.

The drained spin column was removed and the clear buffer was emptied. A new 1.0 mL collection tube was placed in the bottom of the 15 mL centrifuge tube. 100 µL of the neutralized reaction mixture containing the cDNA was loaded directly into the center of the spin column media. The spin column assembly was centrifuged at 10,000x g for about 2.5 minutes upon reaching full acceleration. The eluate collected in the new collection tube was then recovered. The original volume +/-10 percent was recovered. The eluate comprised the cDNA probe.

### Hybridization of cDNA/Dendrimer Probe Mixture to Microarray

The DNA hybridization buffer was thawed and resuspended by heating to 65°C and maintained at 65°C for ten (10) minutes. The hybridization buffer comprised of 40% formamide. The buffer was mixed by inversion to ensure that the components were resuspended evenly. The heating and mixing was repeated until all the material was resuspended. A quantity of competitor DNA (e.g. COT-1-DNA, and polydA) may be added, if required. 5.0 to 10 µL of the eluted cDNA probe was used for hybridization. In a first embodiment, single channel analysis, 2.5 µL of one type of 3DNA® reagent (Genisphere, Inc., Montvale, NJ) (Cy3 or Cy5) was added to the resuspended cDNA along with 12.5 µL of a DNA hybridization buffer (containing 40% formamide). In an alternative embodiment, for dual channel analysis, 2.5 µL of two types of 3DNA® reagents, Cy3 and Cy5 specifically labeled dendrimers, were added to the resuspended cDNA along with 10 µl of a DNA hybridization buffer. In a further embodiment of multiple channel analysis (with three or more channels), 2.5 µL of three or more types of 3DNA® reagents, Cy3, Cy5, and one or more prepared using another label moiety, were added to the resuspended cDNA along with 10µL of a DNA hybridization buffer.

For larger hybridization buffer volumes, additional amounts of the DNA hybridization buffer may be added to reach the required final volume. It is also noted that hybridization buffer volumes greater than 35 µL may also require additional 3DNA® reagents. The DNA hybridization buffer mixture was incubated at a temperature of about 50°C for about 15 to 20 minutes to allow for the prehybridization of the cDNA to the 3DNA® reagents or dendrimer probes. At this stage, the dendrimer probes of the 3DNA® reagent hybridized with the capture sequence on the cDNA. After 20 minutes, the DNA hybridization buffer was then added to the microarray. The microarray and the DNA hybridization buffer were covered and incubated overnight in a humidified chamber at a temperature of about 55°C. At this stage, the cDNA was hybridized to the gene probes.

### Post Hybridization Wash

The microarray was briefly washed to remove any excess dendrimer probes. First, the microarray was washed for 10 minutes at 55° C with 2X SSC buffer, containing 0.2%SDS. Then, the microarray was washed for 10 minutes at room temperature with 2X SSC buffer. Finally, the microarray was washed for 10 minutes at room temperature with 0.2X SSC buffer.

### Signal Detection

The microarray was then scanned as directed by the scanner's manufacturer for detecting, analyzing, and assaying the hybridization pattern.

### EXAMPLE 4

### Method for Detection and Assay on a Microarray Using A Detection Kit for cDNA Arrays

### Kit Contents:

| | |
|---|---|
| Vial 1 | Cy3® 3DNA® Reagent (Genisphere, Montvale, NJ). Use at 2.5 µL per 20 µL assay. |
| Vial 2 | Hybridization buffer- 0.25 M NaPO₄, 4.5% SDS, 1 mM EDTA, and 1X SSC. (Stored at -20°C in the dark.) |

### Microarray preparation

A microarray was prepared as directed by the manufacturer or by customary protocol procedures. The nucleic acid sequences comprising the DNA or gene probes were amplified using known techniques in polymerase chain reaction, then spotted onto glass slides, and processed according to conventional procedures.

### 3DNA® Hybridization

The hybridization buffer of Vial 2 was thawed and resuspended by heating to 65°C for 10 minutes. The buffer was mixed by inversion to ensure that the components are resuspended evenly. If necessary the heating and mixing was repeated until all the components have resuspended. 2.5 µL of 3DNA® reagent of Vial 1 was added to 17.5 µL of hybridization buffer to yield a hybridization mixture. The hybridization mixture was added to the microarray. The microarray was covered and incubated at a temperature of from about 37 to 42°C for about 6 hours to overnight in a humidified chamber.

### Post-Hybrdization Wash

The microarray was washed for 10 minutes at 42°C with 2X SSC buffer containing 0.2% SDS. The microarray was then washed for 10 minutes at room temperature with 2X SSC buffer. The microarray was then washed for 10 minutes at room temperature with 0.2X SSC buffer.

### Signal Detection

The microarray was then scanned as directed by the scanner's manufacturer for detecting, analyzing, and assaying the hybridization pattern.

## Claims

1. A method for detection and assay on a microarray, said method comprising the steps of:
1) contacting a microarray having thereon a plurality of features each comprising a particular first nucleotide sequence with a mixture comprising:
a) a first component comprising a cDNA reagent obtained from mRNA of a target sample, said cDNA having a capture sequence; and
b) a second component comprising a dendrimer having at least one first arm comprising a label capable of emitting a detectable signal and at least one second arm having a second nucleotide sequence complementary to the capture sequence;
2) mixing the first and second components at a temperature and for a time sufficient to enable the first component to bind to the second component; and
3) incubating this mixture with said microarray to enable the first nucleotide sequence to bind to the first component, wherein such binding results in the feature emitting the detectable signal.

2. The method of claim 1 further comprising the step of forming the first component comprising the cDNA reagent by contacting the target sample mRNA with a quantity of a RT primer having the capture sequence, a reverse transcriptase, and nucleotides under conditions sufficient for initiating reverse transcription of said mRNA Into the cDNA reagent.

3. The method of claim 2 further comprising the step of purging excess unhybridized RT primer from said first component prior to incubation of said mixture.

4. The method of claim 3 wherein the purging step further comprises the step of passing the first component through a spin column media.

5. The method of any of claims 1 to 4 wherein the temperature sufficient to enable the first component to bind to the second component is from about 50 to 55°C.

6. The method of any of claims 1 to 5 wherein the temperature sufficient to enable the first component to bind to the first nucleotide sequence is from 42 to 60°C.

7. The method of any of claims 1 to 6 wherein the time sufficient to enable the first component to bind to the first nucleotide sequence is from about 4 to greater than 72 hours.

8. The method of any of claims 1 to 7 wherein the time sufficient to enable the second component to bind to the first component is from about 0.25 to 1 hour.

9. The method of claim 6 wherein the microarray and the mixture are incubated overnight at the temperature from about 42 to 60°C in a humidified chamber.

10. The method of any of claims 1 to 9, further comprising scanning the microarray for detecting the detectable signal and the hybridization pattern generated.

11. The method of any of claims 1 to 10, further comprising washing the microarray to purge dendrimers unattached to microarray after the incubation of the microarray and the mixture.

12. The method of claim 11, wherein the washing step further comprises:
washing the microarray with 2X SSC buffer containing 0.2% SDS at 55°C for about 10 minutes;
washing the microarray with 2X SSC buffer at about room temperature for about 10 minutes; and
washing the microarray with 0.2X SSC buffer at about room temperature for about 10 minutes.

13. The method of any of claims 1 to 12, wherein the mixture further comprises a hybridization buffer.

14. The method of claim 13, wherein the hybridization buffer further comprises 0.25 M NaPO₄, 4.5% SDS, 1 mM- EDTA, and 1X SSC.

15. The method of claim 13. wherein the hybridization buffer further comprises 40% formamide, 4X SSC, and 1% SDS.

16. The method of claim 3 wherein the purging step further comprises the use of a hybridization chamber.

17. The method of claim 3 wherein the purging step further comprises the use of a hybridization station.

18. A method for detection and assay on a microarray, said method comprising the steps of:
1) incubating a mixture including:
i) a first component comprising a cDNA reagent obtained from mRNA of a target sample, said cDNA having a capture sequence; and
ii) a second component comprising a dendrimer having at least one first arm comprising a label capable of emitting a detectable signal and at least one second arm having a second nucleotide sequence complementary to the capture sequence,
at a first temperature and for a time sufficient to induce the first component to bind to the second component and form a prehybridized cDNA-dendrimer complex;
2) contacting a microarray having thereon a plurality of features each comprising a particular first nucleotide sequence with said mixture; and
3) incubating the microarray and the prehybridized cDNA-dendrimer complex at a second temperature and for a time sufficient to induce the prehybridized cDNA-dendrimer complex to bind to the first nucleotide sequence, wherein such binding results in the feature emitting the detectable signal whereby a hybridization pattern is generated on the microarray.

19. The method of Claim 18, wherein said cDNA is obtained using a spin column.

20. A method as claimed in any of the preceding claims, wherein said method comprises an assay using dual channel analysis.

21. A method as claimed in any of claims 1-19, wherein said method comprises an assay using multiple channel analysis, said multiple channel analysis using at least three channels.

## Patentansprüche

1. Methode zum Nachweis und zur Bestimmung auf einem Microarray, die Methode umfassend die folgenden Schritte:
(1) in Kontakt bringen eines Microarrays, der eine Mehrzahl von Bereichen hat, welche jeweils eine spezielle erste Nucleotidsequenz umfassen, mit einer Mischung enthaltend:
(a) eine erste Komponente enthaltend ein cDNA-Reagenz, erhalten aus mRNA einer Zielprobe, wobei die cDNA eine Fang-Sequenz aufweist; und
(b) eine zweite Komponente enthaltend einen Dendrimer, der mindestens einen ersten Arm aufweist, welcher seinerseits eine Markierung aufweist, welche fähig ist, ein detektierbares Signal abzugeben, wobei der Dendrimer mindestens einen zweiten Arm aufweist, der eine zweite Nucleotidsequenz zeigt, welche komplementär zu der Fang-Sequenz ist;
(2) vermischen der ersten und der zweiten Komponente, bei einer Temperatur und für eine Dauer, die ausreichend ist um das Binden der ersten Komponente and die zweite Komponente zu ermöglichen; und
(3) Inkubieren dieser Mischung mit besagtem Microarray, um das Binden der ersten Nucletidsequenz an die erste Komponente zu ermöglichen, wobei eine solche Bindung dazu führt, dass der Bereich ein detektierbares Signal aussendet.

2. Methode nach Anspruch 1, weiterhin umfassend den Schritt der Herstellung der ersten Komponente, enthaltend das cDNA-Reagenz, durch in Kontakt bringen der Zielproben-mRNA mit einer Menge RT Primer enthaltend die Fang-Sequenz, eine reverse Transkriptase und Nucletide, unter Bedingungen, die eine Initiierung der reversen Transkription besagter mRNA in das cDNA-Reagenz erlauben.

3. Die Methode nach Anspruch 2, weiterhin umfassend den Schritt des Entfemens des überschüssigen RT Primers aus besagter ersten Komponente vor dem Inkubieren besagter Mischung.

4. Methode nach Anspruch 3, wobei der Schritt des Entfemens den Schritt des Hindurchführens der ersten Komponente durch ein Zentrifugiersäulenmedium umfasst.

5. Methode nach einem der Ansprüche 1 bis 4, wobei die Temperatur, die ausreichend ist um das Binden der ersten Komponente and die zweite Komponente zu ermöglichen, ungefähr 50 bis 55°C ist.

6. Methode nach einem der Ansprüche 1 bis 5, wobei die Temperatur, welche ein Binden der ersten Komponente and die erste Nukleotidsequenz erlaubt, 42 bis 60°C ist.

7. Methode nach einem der Ansprüche 1 bis 6, wobei Zeit, welche ein Binden der ersten Komponente and die erste Nukleotidsequenz erlaubt, ungefähr 4 bis mehr als 72 Stunden ist.

8. Methode nach einem der Ansprüche 1 bis 7, wobei wobei die Zeit, die ausreichend ist um das Binden der zweiten Komponente and die erste Komponente zu ermöglichen, ungefähr 0.25 bis 1 Stunde ist.

9. Methode nach Anspruch 6, wobei der Microarray und die Mischung über Nacht bei einer Temperatur von ungefähr 42 bis 60°C in einer feuchten Kammer inkubiert werden.

10. Methode nach einem der Ansprüche 1 bis 9, bei der weiterhin der Microarray ausgelesen wird, um das detektierbare Signal und das entstandene Hybridisierungsmuster zu detektieren.

11. Methode nach einem der Ansprüche 1 bis 10, bei der zusätzlich der Microarray gewaschen wird, um nach der Inkubation des Microarrays und der Mischung nicht anhaftende Dendrimere zu entfernen.

12. Methode nach Anspruch 11, wobei der Wasch-Schritt zusätzlich beinhaltet:
Waschen des Microarrays mit 2X SSC Puffer enthaltend 0.2% SDS bei 55°C für ungefähr 10 Minuten;
Waschen des Microarrays mit 2X SSC Puffer bei ungefähr Raumtemperatur für ungefähr 10 Minuten; und
Waschen des Microarrays mit 0.2X SSC Puffer bei ungefähr Raumtemperatur für ungefähr 10 Minuten.

13. Methode nach einem der Ansprüche 1 bis 12, wobei die Mischung weiterhin einen Hybridisierungspuffer enthält.

14. Methode nach Anspruch 13, wobei der Hybridisierungspuffer weiterhin 0.25 M NaPO₄, 4.5% SDS, 1 mM EDTA und 1X SSC enthält.

15. Methode nach Anspruch 13, wobei der Hybridisierungspuffer weiterhin 40% formamide, 4X SSC und 1% SDS enthält.

16. Methode nach Anspruch 3, wobei der Schritt des Entfernens weiterhin die Verwendung einer Hybridisierungskammer beinhaltet.

17. Methode nach Anspruch 3, wobei der Schritt des Entfernens weiterhin die Verwendung einer Hybridisierungsstation beinhaltet.

18. Methode zum Nachweis und zur Bestimmung auf einem Microarray, die Methode umfassend die folgenden Schritte:
(1) Inkubieren einer Mischung enthaltend:
i) eine erste Komponente enthaltend ein cDNA-Reagenz, welches aus mRNA einer Zielprobe erhalten wurde, wobei die cDNA eine Fang-Sequenz aufweist; und
ii) eine zweite Komponente enthaltend einen Dendrimer, der mindestens einen ersten Arm aufweist, welcher seinerseits eine Markierung aufweist, welche fähig ist, ein detektierbares Signal abzugeben, wobei der Dendrimer mindestens einen zweiten Arm aufweist, der eine zweite Nucleotidsequenz zeigt, welche komplementär zu der Fang-Sequenz ist,
bei einer ersten Temperatur und für eine Zeit, die ausreicht, um eine Bindung der ersten Komponente an die zweite Komponente zu veranlassen, und dabei einen vor-hybridisierten cDNA-Dendrimer-Komplex zu bilden; und
(2) in Kontakt bringen eines Microarrays, der eine Mehrzahl von Bereichen hat, welche jeweils eine erste spezielle erste Nucleotidsequenz umfassen, mit besagter Mischung; und
(3) Inkubieren des Microarrays und dem vor-hybridisierten cDNA-Dendrimer-Komplex bei einer zweiten Temperatur und für eine Zeit, die ausreicht, um ein Binden des vor-hybridisierten cDNA-Dendrimer-Komplexes an die erste Nucleotidsequenz zu veranlassen, wobei das Binden dazu führt, dass der Bereich ein detektierbares Signal aussendet, wodurch ein Hybridisierungsmuster auf dem Microarray erzeugt wird.

19. Methode nach Anspruch 18, wobei besagte cDNA mit Hilfe einer Zentrifugensäule erhalten wird.

20. Methode nach einem der vorhergehenden Ansprüche, wobei besagte Methode eine Bestimmungsverfahren beinhaltet, welches sich der Zweikanal-Analyse bedient.

21. Eine Methode nach einem der Ansprüche 1-19, wobei besagte Methode ein Bestimmungsverfahren beinhaltet, welches sich der Mehrkanal-Analyse bedient, wobei besagte Mehrkanalanalyse mindestens drei Kanäle verwendet.

## Revendications

1. Procédé de détection et d'essai sur un microréseau, ledit procédé comprenant les étapes de :
1) mise en contact d'un microréseau sur lequel se trouvent une pluralité d'éléments comprenant chacun une première séquence nucléotidique particulière avec un mélange comprenant :
a) un premier composant comprenant un réactif ADNc obtenu à partir d'ARNm d'un échantillon simple, ledit ADNc ayant une séquence de capture ; et
b) un second composant comprenant un dendrimère ayant au moins un premier bras comprenant un marqueur capable d'émettre un signal détectable et au moins un second bras ayant une seconde séquence nucléotidique complémentaire de la séquence de capture ;
2) mélange des premier et second composants à une température et pendant une durée suffisantes pour permettre au premier composant de se lier au second composant ; et
3) incubation de ce mélange avec ledit microréseau pour permettre à la première séquence nucléotidique de se lier au premier composant, où une telle liaison conduit au fait que l'élément émet le signal détectable.

2. Procédé selon la revendication 1 comprenant en outre l'étape de formation du premier composant comprenant le réactif ADNc par mise en contact de l'ARNm de l'échantillon cible avec une quantité d'amorce TI ayant la séquence de capture, une transcriptase inverse, et des nucléotides dans des conditions suffisantes pour initier la transcription inverse dudit ARNm en le réactif ADNc.

3. Procédé selon la revendication 2 comprenant en outre l'étape d'élimination de l'amorce TI non hybridée en excès dudit premier composant avant l'incubation dudit mélange.

4. Procédé selon la revendication 3 où l'étape d'élimination comprend en outre l'étape de passage du premier composant dans un milieu à colonne tournante.

5. Procédé selon l'une quelconque des revendications 1 à 4 où la température suffisante pour permettre au premier composant de se lier au second composant est d'environ 50 à 55°C.

6. Procédé selon l'une quelconque des revendications 1 à 5 où la température suffisante pour permettre au premier composant de se lier à la première séquence nucléotidique est de 42 à 60°C.

7. Procédé selon l'une quelconque des revendications 1 à 6 où la durée suffisante pour permettre au premier composant de se lier à la première séquence nucléotidique est d'environ 4 à plus de 72 h.

8. Procédé selon l'une quelconque des revendications 1 à 7 où la durée suffisante pour permettre au second composant de se lier au premier composant est d'environ 0,25 à 1 h.

9. Procédé selon la revendication 6 où le microréseau et le mélange sont incubés pendant une nuit à la température d'environ 42 à 60°C dans une chambre humidifiée.

10. Procédé selon l'une quelconque des revendications 1, à 9 comprenant en outre le balayage du microréseau pour détecter le signal détectable et la configuration d'hybridation produite.

11. Procédé selon l'une quelconque des revendications 1, à 10 comprenant en outre le lavage du microréseau pour éliminer les dendrimères non fixés au microréseau après l'incubation du microréseau et du mélange.

12. Procédé selon la revendication 11 où l'étape de lavage comprend en outre :
un lavage du microréseau avec du tampon 2X SSC contenant 0,2 % de SDS à 55°C pendant environ 10 min ;
un lavage du microréseau avec du tampon 2X SSC sensiblement à la température ambiante pendant environ 10 min ; et
un lavage du microréseau avec du tampon 0,2X SSC sensiblement à la température ambiante pendant environ 10 min.

13. Procédé selon l'une quelconque des revendications 1 à 12 où le mélange comprend en outre un tampon d'hybridation.

14. Procédé selon la revendication 13 où le tampon d'hybridation comprend en outre 0,25 M de NaPO₄, 4,5 % de SDS, 1 mM de EDTA et 1X SSC.

15. Procédé selon la revendication 13 où le tampon d'hybridation comprend en outre 40 % de formamide, 4X SSC et 1 % de SDS.

16. Procédé selon la revendication 3 où l'étape d'élimination comprend en outre l'utilisation d'une chambre d'hybridation.

17. Procédé selon la revendication 3 où l'étape d'élimination comprend en outre l'utilisation d'un poste d'hybridation.

18. Procédé de détection et d'essai sur un microréseau, ledit procédé comprenant les étapes de :
1) incubation d'un mélange incluant :
i) un premier composant comprenant un réactif ADNc obtenu à partir d'ARNm d'un échantillon cible, ledit ADNc ayant une séquence de capture ; et
ii) un second composant comprenant un dendrimère ayant au moins un premier bras comprenant un marqueur capable d'émettre un signal détectable et au moins un second bras ayant une seconde séquence nucléotidique complémentaire de la séquence de capture,
à une première température et pendant une durée suffisantes pour amener le premier composant à se lier au second composant et former un complexe ADNc-dendrimère préhybridé ;
2) mise en contact d'un microréseau sur lequel se trouvent une pluralité d'éléments comprenant chacun une première séquence nudéotidique particulière avec ledit mélange ; et
3) incubation du microréseau et du complexe ADNc-dendrimère préhybridé à une seconde température et pendant une durée suffisantes pour amener le complexe ADNc-dendrimère préhybridé à se lier à la première séquence nucléotidique, où une telle liaison conduit au fait que l'élément émet le signal détectable de sorte qu'une configuration d'hybridation est produite sur le microréseau.

19. Procédé selon la revendication 18 où ledit ADNc est obtenu au moyen d'une colonne tournante.

20. Procédé selon l'une quelconque des revendications précédentes où ledit procédé comprend un essai utilisant une analyse à deux canaux.

21. Procédé selon l'une quelconque des revendications 1-19 où ledit procédé comprend un essai utilisant une analyse à canaux multiples, ladite analyse à canaux multiples utilisant au moins trois canaux.
